# EUROPEAN PATENT APPLICATION

(11) **EP 2 924 001 A1**
(43) Date of publication of application: **30.09.2015**
(21) Application number: 13856359.8
(22) Date of filing: 25.11.2013
(51) Int. Cl.: C01B 3/06, A61K 33/00, C01B 3/00, C01B 3/08, C01B 3/56

(54) **HIGH-CONCENTRATION HYDROGEN GAS SUPPLY DEVICE FOR LIVING ORGANISMS**

(30) Priority: 26.11.2012 JP 2012257082; 28.01.2013 JP 2013012881
(71) Applicant: MIZ Co., Ltd., Kamakura-shi, Kanagawa 247-0056 (JP)
(72) Inventor: SATOH, Fumitake, Fujisawa-shi Kanagawa 251-0871 (JP); KUROKAWA, Ryousuke, Fujisawa-shi Kanagawa 251-0871 (JP); SATOH, Bunpei, Fujisawa-shi Kanagawa 251-0871 (JP); SEO, Tomoki, Fujisawa-shi Kanagawa 251-0871 (JP)
(74) Representative: Ter Meer Steinmeister & Partner
(86) International application number: PCT/JP2013/081593
(87) International publication number: WO 2014/081026

(57) **Abstract**

An apparatus includes: a hydrogen generating agent (1); a hydrogen gas generator (3) having the hydrogen generating agent; and at least one of a diluent gas supplier (7) or a vacuum pump (8) for diluting hydrogen gas generated by a reaction of the hydrogen generating agent and water (2) for reaction. The apparatus has a feature that a part or whole of a gas phase portion (4) in the hydrogen gas generator is covered with a hydrogen gas permeable membrane (5), and is operable to: mix the hydrogen gas with the diluent gas supplied from the diluent gas supplier or the vacuum pump thereby to supply a living organism with a mixed gas at a flow volume of 1 mL/min or more, the mixed gas having a hydrogen gas concentration of 0.1 vol% or higher and lower than 18.3 vol%; and maintain a hydrogen gas concentration in a position separated by 7 cm from the hydrogen generating agent or a water surface of the water for reaction at lower than 18.3 vol% during hydrogen generation even when supply of the diluent gas is stopped.

## Description

### [Technical Field]

The present invention relates to an apparatus for supplying high-concentration hydrogen gas for a living organism.

### [Background Art]

There is known an apparatus for supplying hydrogen gas for a living organism configured such that an air mixer is attached to a part of a conduit pipe from a hydrogen gas generator to a nasal cavity cannula and the concentration of hydrogen gas for supply can be arbitrarily set (Patent Document 1).

### [Prior Art Document]

### [Patent Document]

[Patent Document 1] JP 2009-5881 A

### [Summary of Invention]

### [Problems to be solved by Invention]

According to the above conventional apparatus for supplying hydrogen gas for a living organism, however, if the air mixer breaks down and the hydrogen gas cannot be diluted, there is a considerable risk that the hydrogen gas concentration increases continuously in the apparatus. In particular, when a hydrogen generating agent is used as the hydrogen generating source, special attention has to be paid because the reaction of hydrogen generation is difficult to stop after once starting.

Problems to be solved by the present invention include providing an apparatus for supplying high-concentration hydrogen gas for a living organism by which hydrogen gas having health benefits can be used safely in medical practice or at home.

### [Means for solving problems]

The present invention solves the above problems through providing an apparatus for supplying high-concentration hydrogen gas for a living organism. The apparatus maintains the hydrogen gas concentration in a gas phase portion in a hydrogen gas generator at lower than 18.3 vol% through reacting a hydrogen generating agent with water for reaction thereby to generate hydrogen gas and partially discharging the hydrogen gas in the gas phase portion in the hydrogen gas generator to outside of the gas phase portion in the hydrogen gas generator. The apparatus can thereby maintain the hydrogen gas concentration at lower than 18.3 vol%, which is the lower limit of detonation, in all the course from a time when the hydrogen gas is generated to a time when the hydrogen gas is supplied to a living organism.

### [Effect of Invention]

According to the present invention, hydrogen gas having health benefits can be used safely in medical practice or at home.

### [Brief Description of Drawing(s)]

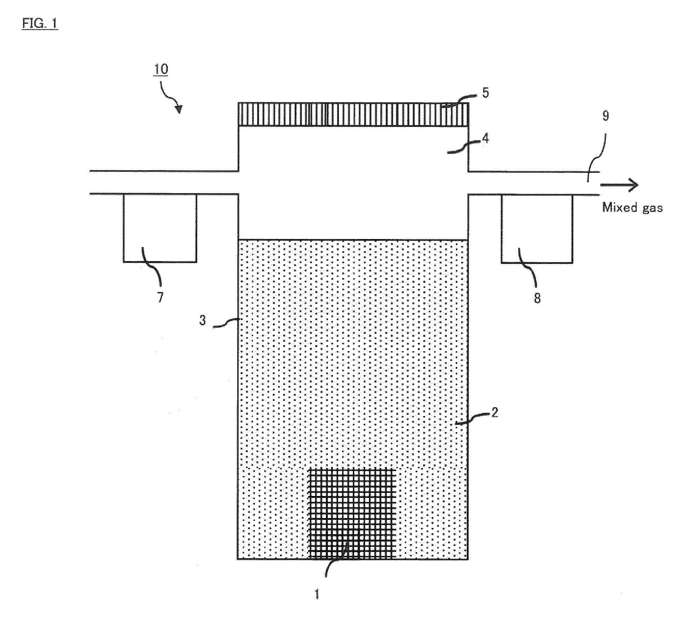

[Fig. 1] FIG. 1 is a diagram illustrating an apparatus for supplying hydrogen gas for a living organism according to an embodiment of the present invention.

### [Mode(s) for Carrying out the Invention]

As shown in FIG. 1, an apparatus 10 for supplying high-concentration hydrogen gas for a living organism according to an embodiment of the present invention includes: a hydrogen generating agent 1; and a hydrogen gas generator 3 that houses the hydrogen generating agent 1 and reacts the hydrogen generating agent 1 with water 2 for reaction thereby to generate hydrogen gas. A part or whole of a gas phase portion 4 in the hydrogen gas generator 3 is covered with a hydrogen gas permeable membrane 5. Therefore, hydrogen gas in the gas phase portion 4 in the hydrogen gas generator 3 is partially discharged outside the hydrogen gas generator 3 via the hydrogen gas permeable membrane 5. Through this operation, the present invention is carried out as the apparatus 10 in which the hydrogen gas concentration in the gas phase portion 4 in the hydrogen gas generator 3 can be maintained at lower than 18.3 vol% and the hydrogen gas concentration in the vicinity of the above of the hydrogen gas permeable membrane 5 can be maintained at lower than 15.0 vol%.

In an alternative embodiment, such an apparatus 10 for supplying high-concentration hydrogen gas for a living organism may further include at least one of a diluent gas supplier 7 or a vacuum pump 8 for diluting the hydrogen gas generated in the gas phase portion 4 in the hydrogen gas generator 3 and for serving an appropriate flow volume of the hydrogen gas to an living organism. According to this configuration, the hydrogen gas is mixed with a diluent gas supplied from the diluent gas supplier 7 or with a diluent gas introduced by the vacuum pump 8 from outside the gas phase portion 4 in the hydrogen gas generator 3 via the hydrogen gas permeable membrane 5. Through this operation, the present invention is carried out as the apparatus 10 by which a living organism can be supplied with a mixed gas that includes the hydrogen gas and the diluent gas and has a hydrogen concentration of lower than 15.0 vol%.

Here, the apparatus 10 for supplying high-concentration hydrogen gas for a living organism is to supply hydrogen gas to a living organism mainly for the purpose of health maintenance and/or functional maintenance of living organisms (including cells and organs), disease improvement and/or functional improvement, or health check and/or functional measurement. Examples of the supply means thereof may include, but are not limited to, supply by way of inhalation from the nasal cavity and/or mouth cavity, supply by way of exposure of and/or blowing to the cells, skin or organ, supply by way of exposure of and/or blowing to a living organism applicable liquid, such as liquid drug and organ storage liquid, which is assumed to be applied to a living organism, and supply by way of diffusion from the outside of a container or circuit which is provided with a living organism.

The hydrogen generating agent 1 as used herein is a substance that generates hydrogen. Examples of the hydrogen generating agent include substances that generate hydrogen by contact with water, such as metals having a higher ionization tendency than that of hydrogen and hydrogenated compounds including a hydride of a metal or a hydride of a metalloid, e.g., a boron hydride compound. In consideration of the safety of the reaction product, the temperature caused by the reaction heat and other factors, it is preferred to use magnesium, aluminum, magnesium hydride, or sodium boron hydride. Among them, it is preferred to use aluminum, which is a metal having a higher ionization tendency than that of hydrogen, magnesium hydride, which is a hydride of a metal, or sodium boron hydride, which is a boron hydride compound, in view of good appearance and stability in hydrogen generation.

Such a hydrogen generating agent 1 may be used in combination with a pH adjuster, such as acid and alkali, as necessary. The hydrogen generating agent 1 may be surrounded by a porous separator, such as a nonwoven fabric, together with the pH adjuster or solely. Examples of such acid include, but are not limited to, phosphates. Examples of such alkali include, but are not limited to, bicarbonates.

The water 2 for reaction is a substance that generates hydrogen gas in the hydrogen gas generator 3 through contact with the hydrogen generating agent 1. Examples of such water 2 for reaction include, but are not limited to, tap water, clean water, ion-exchanged water, purified water, pure water, RO water, and the like. Regardless of the contained components, hardness and liquid properties, substances that contain water fall within the concept of the water for reaction according to the present invention.

The hydrogen gas generated in the hydrogen gas generator 3 via the reaction of the hydrogen generating agent 1 with the water 2 for reaction transfers to the gas phase portion 4 in the hydrogen gas generator 3. Here, when the hydrogen gas generator 3 is closed such as by a cover to avoid diffusion of the hydrogen gas into the air, the hydrogen concentration in the gas phase portion 4 increases as time passes and will exceed 18.3 vol% which is the lower limit of detonation of hydrogen gas.

On the other hand, when the hydrogen gas generator 3 is completely opened to the outside so that the hydrogen gas diffuses into the air immediately after being generated, a sufficient amount of the hydrogen gas cannot be served to a living organism.

Therefore, the hydrogen gas generator 3 according to the present invention has a feature that a part or whole of the gas phase portion 4 in the hydrogen gas generator 3 is covered by the hydrogen gas permeable membrane 5. According to such a configuration, only a part of the hydrogen gas in the gas phase portion 4 in the hydrogen gas generator 3 can be discharged outside the hydrogen gas generator 3 thereby to constantly maintain the hydrogen gas concentration in the gas phase portion 4 in the hydrogen gas generator 3 within a safe range. Such a hydrogen gas permeable membrane 5 may preferably be, but is not limited to, a gas membrane that is poorly-permeable or non-permeable for water but permeable for hydrogen gas.

In particular, when a hydrogen generating agent is used as the hydrogen generating source, the chemical reaction (reaction of hydrogen generation) is difficult to stop after once starting. Therefore, if the supply of the diluent gas is stopped, there is a risk in the prior art that the hydrogen concentration automatically continues to increase to a dangerous range. Even when the hydrogen gas generator is in a state of being half opened rather than in a completely closed system, if a sufficient space is not ensured in the hydrogen gas generator or in a partition that houses the hydrogen generating agent, the generated hydrogen gas cannot be sufficiently discharged, so that there is a concern that the hydrogen concentration increases to a dangerous range, even locally, at some close spot approaching the hydrogen generating agent which is reacting to generate hydrogen.

In contrast, according to the feature of the present invention, the generated hydrogen gas does not stay at a local space even if the supply of the diluent gas to the hydrogen gas generator is stopped. This allows the apparatus to be left safely until the reaction of hydrogen generation is completed.

To further enhance the safety, it is preferred that a use amount of the hydrogen generating agent is less than {(flow volume (L/min) × 0.183 × estimated time (min) of continuous use) ÷ (substance quantity (mol) of hydrogen molecules generated from 1 mol of the hydrogen generating agent in a chemical reaction formula of the hydrogen generating agent and water × 22.4)} mol, as will be described later.

For the same reason, the hydrogen gas generator may have a volume twice or more that of the hydrogen generating agent to be used, preferably 3 times or more, and more preferably 5 times or more. Here, in an apparatus in which the outline of the hydrogen gas generator is difficult to be definitely defined because the hydrogen gas generator is not completely closed and is partially opened, the volume of a part that can be measured from back and forth as a space that houses the hydrogen generating agent may be provisionally deemed as a "volume of the hydrogen gas generator," such as using a drawing of the apparatus.

In general, it is said that hydrogen reacts with oxygen in the air to become detonating gas when the hydrogen concentration exceeds 4.7 vol%. However, an event that such detonating gas leads to actual ignition (deflagration) may occur in most situations at a concentration much higher than 4.7 vol% which is said to be the explosion limit of hydrogen. According to the applicants' experiment, when the hydrogen concentration exceeds 10 to 15 vol%, the detonation is offensive to the ear to some extent and small deflagration occurs.

However, even though a high concentration of hydrogen is intended to be obtained, it may be inappropriate to require a concentration of hydrogen gas higher than 18.3% (lower limit of detonation) that is a concentration at which the reaction of hydrogen and oxygen causes a shock wave to propagate as detonation around there.

That is, according to the present invention, the hydrogen gas concentration in the gas phase portion 4 in the hydrogen gas generator 3 may preferably be maintained at lower than 18.3 vol%, more preferably lower than 15.0 vol%, and further preferably lower than 4.7 vol%.

Similarly, the hydrogen gas concentration in the vicinity of the hydrogen gas permeable membrane 5 of the hydrogen gas generator 3 may preferably be maintained at lower than 15.0 vol%, more preferably lower than 10.0 vol%, and further preferably lower than 3.0 vol%.

It is also preferred that the apparatus 10 for supplying high-concentration hydrogen gas for a living organism according to the present invention further comprises at least one of a diluent gas supplier 7 or a vacuum pump 8. The hydrogen gas in the gas phase portion 4 in the hydrogen gas generator 3 is mixed with the diluent gas supplied via the diluent gas supplier 7 and/or the vacuum pump 8 which may be provided at any point along the path through which the hydrogen gas is served to a living organism. Through this operation, a flow volume can be ensured at which the hydrogen is more efficiently carried, and the mixed gas containing the hydrogen gas and the diluent gas can be supplied to a living organism at a more preferred concentration lower than 15.0 vol%, or at a further preferred concentration lower than 10.0 vol%, or at a furthermore preferred concentration lower than 4.7 vol%.

It is preferred that the supply of the diluent gas is started as soon as possible after the hydrogen generating agent 1 is contacted with the water 2 for reaction, or started prior to contact with the water 2 for reaction.

The diluent gas according to the present invention refers to a concept that includes normal air and artificial air, and examples thereof include medical gas of which the oxygen concentration is adjusted and other medical gases which contain medical components such as anesthetic component. Examples of the diluent gas supplier 7 which supplies such diluent gas include an apparatus, such as an air pump, which can blow the diluent gas.

The above-described vicinity of the hydrogen gas permeable membrane 5 of the hydrogen gas generator 3 refers to a concept that includes a position separated by 7 cm and preferably 3 cm from the hydrogen gas permeable membrane 5, and most preferably a position in contact with the above of the hydrogen gas permeable membrane 5. Generally, the hydrogen gas concentration can be understood to increase as the measurement point comes close to the hydrogen gas permeable membrane 5. Therefore, when the hydrogen gas concentration at a position separated by 7 cm from the hydrogen gas permeable membrane 5 is lower than 18.3 vol%, the hydrogen gas concentration at a closer range than 7 cm can be deemed also to be lower than 18.3 vol%. Accordingly, if the measurement at the above position is difficult due to the size of the apparatus 10 for supplying high-concentration hydrogen gas for a living organism or other reason, the measurement may be performed at a closer range than the above position but at a position as close as possible to the above position.

In the present invention, the hydrogen gas concentration in the vicinity of the hydrogen gas permeable membrane 5 of the hydrogen gas generator 3 is measured every 1 minute during 10 minutes from the hydrogen generation and thereafter every 10 minutes for the total of 120 minutes (if the reaction is completed therebefore, measurement is continued until that time), and when the maximum value is lower than 18.3 vol%, it is deemed that "the hydrogen gas concentration in the vicinity of the hydrogen gas permeable membrane of the hydrogen gas generator is maintained at lower than 18.3 vol%."

Likewise, in the present invention, the hydrogen gas concentration in the gas phase portion 4 in the hydrogen gas generator 3 is measured every 1 minute during 10 minutes from the hydrogen generation and thereafter every 10 minutes for the total of 120 minutes (if the reaction is completed therebefore, measurement is continued until that time), and when the maximum value is lower than 18.3 vol%, it is deemed that "the hydrogen gas concentration in the gas phase portion 4 in the hydrogen gas generator is maintained at lower than 18.3 vol%."

The present invention is an invention that relates consistently to the apparatus 10 for supplying high-concentration hydrogen gas for a living organism. Therefore, even though the hydrogen gas concentration is to be maintained lower than 18.3 vol%, the dilution may not have to be needed beyond necessity. It is thus preferred, unlike the handling of hydrogen gas in other industrial fields, to perform management of conditions for the hydrogen generation and/or management of the flow volume rather than aiming to reduce the hydrogen gas concentration close to zero without limit so that the generated hydrogen gas can be safely discarded outside the system.

For example, when the diluent gas is supplied at a flow volume of 5 L/min via the diluent gas supplier 7 and/or the vacuum pump 8, it is preferred that the hydrogen gas is maintained with a generation amount of 0.915 L/min (18.3% of 5 L). Therefore, when continuous use for 120 minutes is expected, the total generation amount of hydrogen is 109.8 L (0.915 L×120 min). Here, if sodium boron hydride is used as the hydrogen generating agent, the reaction formula of sodium boron hydride and water is as follows:

NaBH₄ + 2H₂O → 4H₂ + NaBO₂

Since 4 mol (89.6 L) of hydrogen gas are generated from 1 mol of sodium boron hydride, it is preferred that the sodium boron hydride to be used is less than 1.225 mol (109.8 L÷89.6 L) or 46.3 g (37.83 g/mol× 1.225 mol).

In a similar manner, when metal magnesium is used as substituted for sodium boron hydride, the reaction formula of metal magnesium and water is as follows:

Mg + 2H₂O → Mg(OH)₂ + H₂

Since 1 mol (22.4 L) of hydrogen gas is generated from 1 mol of metal magnesium, it is preferred that the metal magnesium to be used is less than 4.9 mol (109.8 L÷22.4 L) or 117.6 g (24 g/mol×4.9 mol).

The above can be generalized such that a use amount of the hydrogen generating agent in the present invention is preferably less than {(flow volume (L/min) × 0.183 × estimated time (min) of continuous use) ÷ (substance quantity (mol) of hydrogen molecules generated from 1 mol of the hydrogen generating agent in a chemical reaction formula of the hydrogen generating agent and water × 22.4)} mol. In view of the safety, the constant 0.183 in the above expression may preferably be substituted by 0.150 (15%), and more preferably 0.047 (4.7%). When the hydrogen generating agent is used in combination with a pH adjuster, the pH adjuster may appear in the above chemical reaction formula of the hydrogen generating agent and water.

In view of effects to be obtained, it is preferred in the present invention that the hydrogen gas concentration in the mixed gas containing the hydrogen gas and the diluent gas is 0.01 vol% or more, preferably 0.1 vol% or more, and more preferably 1.0 vol% or more.

Therefore, it is preferred that a use amount of the hydrogen generating agent in the present invention is not less than {(flow volume (L/min) × 0.0001 × estimated time (min) of continuous use) ÷ (substance quantity (mol) of hydrogen molecules generated from 1 mol of the hydrogen generating agent in a chemical reaction formula of the hydrogen generating agent and water × 22.4)} mol. The constant 0.0001 in the above expression may preferably be substituted by 0.001 (0.1 %), and more preferably 0.01 (1%).

With regard to the flow volume, in terms of the amount of air or high-concentration oxygen gas which human beings or animals inhale for 1 minute, it is preferred that the diluent gas is blown at a flow volume of 1 mL/min or more, preferably 1 L/min or more, more preferably 2 L/min or more, further preferably 4 L/min or more, and particularly preferably 6 L/min or more, into the hydrogen gas generator via the diluent gas supplier and/or the vacuum pump, for example.

Examples of a form to supply the mixed gas of the hydrogen gas and the diluent gas to a living organism include a form to inhale the mixed gas by moving the face directly to the hydrogen gas permeable membrane 5 of the hydrogen gas generator 3 and a form to inhale the mixed gas from a mixed gas outlet 9 provided at the hydrogen gas generator 3.

Moreover, an attachment such as a nasal cavity cannula may be appropriately connected to the vacuum pump 8 or the mixed gas outlet 9 thereby to enhance the convenience at the time of inhale and/or the stability of supply of the mixed gas.

### [Examples]

Working examples of the present invention will hereinafter be described. Unless otherwise stated in the present application, various meters used to measure various physical property values include a hydrogen gas concentration meter "XP-3140 (available from New Cosmos Electric Co., Ltd)."

### [Example 1]

The hydrogen gas generator was prepared as a plastic container having a volume of 3,500 mL, of which the upper face of a cover part was covered with a hydrogen gas permeable membrane (Tyvek 1073B available from DuPont-Asahi Flash Spun Products Co., Ltd., here and hereinafter). The hydrogen gas generator was provided therein with 27 g of sodium boron hydride (sodium tetrahydroborate, powder, available from Wako Pure Chemical Industries, Ltd., here and hereinafter) as the hydrogen generating agent, 1.5 g of sodium dihydrogen phosphate (sodium dihydrogen phosphate available from Wako Pure Chemical Industries, Ltd., here and hereinafter), and 19.5 g of sodium hydrogen carbonate (sodium bicarbonate available from TOSOH CORPORATION, here and hereinafter), and the hydrogen generating agent was reacted with 500 cc of Fujisawa city tap water as the water for reaction thereby to generate hydrogen gas. The cover part was closed, and the hydrogen gas concentration in the gas phase portion in the hydrogen gas generator was measured every 1 minute during 10 minutes from the hydrogen generation and thereafter every 10 minutes for the total of 120 minutes. The maximum hydrogen gas concentration was recorded. The result is listed in Table 1.

### [Example 2]

The hydrogen gas generator was prepared as a plastic container having a volume of 3,500 mL, of which the upper face of a cover part was covered with the hydrogen gas permeable membrane. The hydrogen gas generator was provided therein with 27 g of the sodium boron hydride as the hydrogen generating agent, 1.5 g of the sodium dihydrogen phosphate, and 19.5 g of the sodium hydrogen carbonate, and the hydrogen generating agent was reacted with 500 cc of Fujisawa city tap water as the water for reaction thereby to generate hydrogen gas, thereafter the cover part was closed. An air pump (Silent β-120 available from Marukan Co., Ltd.) as the diluent gas supplier was provided at a tube led out from the hydrogen gas generator, and normal air was blown therein at a flow volume of 5 L/ min to dilute the hydrogen gas. The hydrogen gas concentration in the gas phase portion in the hydrogen gas generator was measured every 1 minute during 10 minutes from the hydrogen generation and thereafter every 10 minutes for the total of 120 minutes, and the maximum hydrogen gas concentration was recorded. The result is listed in Table 1.

### [Example 3]

The hydrogen gas generator was prepared as a plastic container having a volume of 3,500 mL, of which the upper face of a cover part was covered with the hydrogen gas permeable membrane. The hydrogen gas generator was provided therein with 27 g of the sodium boron hydride as the hydrogen generating agent, 1.5 g of the sodium dihydrogen phosphate, and 19.5 g of the sodium hydrogen carbonate, and the hydrogen generating agent was reacted with 500 cc of Fujisawa city tap water as the water for reaction thereby to generate hydrogen gas. The cover part was closed, and a vacuum pump (vacuum pump, DAP-6D, available from ULVAC KIKO, Inc.) was operated to take in normal air with 7 L/min from outside the hydrogen gas generator so that the normal air would be blown into the hydrogen gas generator. The hydrogen gas concentration in the gas phase portion in the hydrogen gas generator was measured every 1 minute during 10 minutes from the hydrogen generation and thereafter every 10 minutes for the total of 120 minutes, and the maximum hydrogen gas concentration was recorded. The result is listed in Table 1.

### [Example 4]

The hydrogen gas generator was prepared as a plastic container having a volume of 3,500 mL, of which the upper face of a cover part was covered with the hydrogen gas permeable membrane. The hydrogen gas generator was provided therein with 27 g of the sodium boron hydride as the hydrogen generating agent, 1.5 g of the sodium dihydrogen phosphate, and 19.5 g of the sodium hydrogen carbonate, and the hydrogen generating agent was reacted with 500 cc of Fujisawa city tap water as the water for reaction thereby to generate hydrogen gas. The cover part was closed, and an air pump (Silent β-120 available from Marukan Co., Ltd.) was used as the diluent gas supplier to blow normal air into the hydrogen gas generator at a flow volume of 5 L/ min. The hydrogen gas concentration in the gas phase portion in the hydrogen gas generator was measured every 1 minute during 10 minutes from the hydrogen generation and thereafter every 10 minutes for the total of 60 minutes. After 60 minutes elapsed, the air pump was stopped, and subsequently the hydrogen gas concentration in the gas phase portion in the hydrogen gas generator was measured every 1 minute during 10 minutes from the stop of the air pump and thereafter every 10 minutes for the total of 60 minutes.

The maximum hydrogen gas concentration was recorded each for 60 minutes before and after the stop of the air pump. The results are listed in Table 1.

### [Comparative Example 1]

The cover part in Example 1 was changed to a simple cover part having an upper face without being covered by a hydrogen gas permeable membrane. The result is listed in Table 1 (when the measurement was performed after 2 minutes elapsed, there was recorded 24.0 vol% beyond the detonation concentration, and the subsequent experiment was discontinued in view of the safety).

### [Comparative Example 1]

The contents in Example 1 were changed to 47 g of the sodium boron hydride and 10 g of the sodium dihydrogen phosphate (no sodium hydrogen carbonate). The result is listed in Table 1 (when the measurement was performed after 4 minutes elapsed, there was recorded 19.0 vol% beyond the detonation concentration, and the subsequent experiment was discontinued in view of the safety).

**[Table 1]**

| | | Hydrogen gas concentration (vol%) |
|---|---|---|
| Example 1 | | 15 |
| Example 2 | | 9,2 |
| Example 3 | | 6,5 |
| Example 4 | Before | 8,2 |
| | After | 14 |
| Comparative Example 1 | | 24 |
| Comparative Example 2 | | 19 |

### [Description of Reference Numerals]

- 1: Hydrogen generating agent
- 2: Water for reaction
- 3: Hydrogen gas generator
- 4: Gas phase portion in hydrogen gas generator
- 5: Hydrogen gas permeable membrane
- 7: Diluent gas supplier
- 8: Vacuum pump
- 9: Mixed gas outlet
- 10: Apparatus for supplying hydrogen gas for a living organism

## Claims

1. An apparatus for supplying hydrogen gas for a living organism comprising:
a hydrogen generating agent;
a hydrogen gas generator having the hydrogen generating agent; and
at least one of a diluent gas supplier or a vacuum pump for diluting hydrogen gas generated by a reaction of the hydrogen generating agent and water for reaction,
wherein a part or whole of a gas phase portion in the hydrogen gas generator is covered with a hydrogen gas permeable membrane,
the apparatus is operable to:
mix the hydrogen gas with the diluent gas supplied using the diluent gas supplier or the vacuum pump thereby to supply a living organism with a mixed gas at a flow volume of 1 mL/min or more, the mixed gas having a hydrogen gas concentration of 0.1 vol% or higher and lower than 18.3 vol%; and
maintain a hydrogen gas concentration in a position separated by 7 cm from the hydrogen generating agent or a water surface of the water for reaction at lower than 18.3 vol% during hydrogen generation even when supply of the diluent gas is stopped.

2. The apparatus for supplying hydrogen gas for a living organism according to claim 1, wherein the hydrogen generating agent contains at least one of a metal having a higher ionization tendency than that of hydrogen, a hydride of a metal, or a hydride of a metalloid.

3. The apparatus for supplying hydrogen gas for a living organism according to claim 1, wherein the hydrogen generating agent is a boron hydride compound.

4. The apparatus for supplying hydrogen gas for a living organism according to claim 1, wherein the hydrogen generating agent is a magnesium hydride.

5. The apparatus for supplying hydrogen gas for a living organism according to claim 1, wherein the hydrogen generating agent is a metal aluminum.

6. The apparatus for supplying hydrogen gas for a living organism according to claim 1, wherein a use amount of the hydrogen generating agent is less than {(flow volume (L/min) × 0.183 × estimated time (min) of continuous use) ÷ (substance quantity (mol) of hydrogen molecules generated from 1 mol of the hydrogen generating agent in a chemical reaction formula of the hydrogen generating agent and water × 22.4)} mol.

7. The apparatus for supplying hydrogen gas for a living organism according to claim 1, wherein the hydrogen generating agent is surrounded by a nonwoven fabric.

8. The apparatus for supplying hydrogen gas for a living organism according to claim 1, wherein the hydrogen gas generator has a volume twice or more that of the hydrogen generating agent.

9. The apparatus for supplying hydrogen gas for a living organism according to claim 1, wherein the hydrogen concentration in the mixed gas is 0.1 vol% or higher and lower than 15 vol%.

10. The apparatus for supplying hydrogen gas for a living organism according to claim 1, wherein the diluent gas is normal air.
